Europäisches Patentamt

(19) European Patent Office

Office européen des brevets

(11) Veröffentlichungsnummer: **0 046 485**
A1

(12) # EUROPÄISCHE PATENTANMELDUNG

(21) Anmeldenummer: 81104421.3

(51) Int. Cl.³: **A 61 M 25/00**

(22) Anmeldetag: 09.06.81

(30) Priorität: 27.08.80 DE 8022846 U

(43) Veröffentlichungstag der Anmeldung:
03.03.82 Patentblatt 82/9

(84) Benannte Vertragsstaaten:
AT DE FR GB IT SE

(71) Anmelder: SIEMENS AKTIENGESELLSCHAFT Berlin
und München
Postfach 22 02 61
D-8000 München 22(DE)

(72) Erfinder: Funke, Helmut
Ruhsteinstrasse 2
D-8521 Möhrendorf(DE)

(72) Erfinder: Glaser, Friedrich
Luitpoldstrasse 70
D-8520 Erlangen(DE)

(72) Erfinder: Schweikert, Eugen
Sudetenstrasse 34
D-8521 Bubenreuth(DE)

(54) **Katheter.**

(57) Die Erfindung bezieht sich auf einen Katheter zum Durchfluß von Flüssigkeiten, bestehend aus einem Kunststoffschlauch mit wenigstens einem distalen Anschluß. Derartige Katheter dienen beispielsweise zur Verbindung der Dosiereinheit eines am menschlichen Körper tragbaren Infusionsgerätes mit dem Patientenkörper. Gemäß der Erfindung ist der Kunststoffschlauch (1, 101, 201) mit einem weiteren Schlauch (3, 103, 203) als Knickschutz umhüllt. In erster Ausbildung der Erfindung ist lediglich der dem Anschluß (2) zugewandte Bereich des Kunststoffschlauches (1) mit dem weiteren Schlauch (3) umhüllt. In weiteren Ausbildungen der Erfindung verläuft der äußere Schlauch (103) über die Länge des gesamten inneren Katheterschlauches (101) oder ist der äußere Schlauch (203) länger als der innere Katheterschlauch (101).

FIG 1

EP 0 046 485 A1

SIEMENS AKTIENGESELLSCHAFT        Unser Zeichen
Berlin und München                VPA 80 P 5116 E

Katheter

Die Erfindung bezieht sich auf einen Katheter zum Durchfluß von Flüssigkeiten, bestehend aus einem Kunststoffschlauch mit wenigstens einem distalen Anschluß.

Für die betriebsmäßige Verwendung von am oder im Patientenkörper tragbaren Infusionsgeräten werden Katheter benötigt, durch die die Infusionsflüssigkeit von der Förder- und Dosiereinheit des Infusionsgerätes am Patientenkörper gelangt. Solche Katheter zum Anschluß an die Dosiereinheit können so lang ausgebildet sein, daß sie mit ihrem proximalen Ende unmittelbar an den gewünschten Ort zum Ausfluß der Flüssigkeit legbar sind. Andererseits können solche Katheter auch aus mehreren Teilstücken bestehen. In jedem Fall ist es erforderlich, den Katheter zumindest mit seinem rückwärtigen Ende mit einem Anschluß für die Dosiereinheit zu versehen, welche schnell betätigbar ist. Dafür werden üblicherweise sogenannte Luer-Lock-Verbindungen verwendet. Entsprechend weiblich oder männlich ausgebildete Anschlüsse werden als Kunststoffspritzteile gefertigt, wobei der flexible Katheterschlauch bereits beim Herstellungsvorgang dicht mit dem Anschlußteil verbunden werden kann.

Beim Gebrauch solcher Katheter ist üblicherweise der Ansatz des Katheterschlauches am Anschlußstück am meisten beansprucht, so daß er dort leicht abbrechen kann. Daneben können insbesondere Abschnitte von

Wht 5 Rl / 26.05.1981

**0046485**

Kathetern, die sich außerhalb des Patientenkörpers befinden, durch Bewegung des Patienten od.dgl. abknicken und damit den Flüssigkeitsdurchfluß unmöglich machen. Derartige Störungen müssen aber mit Sicherheit beim Betrieb von Infusionsgeräten ausgeschlossen werden.

Aufgabe der Erfindung ist es daher, einen Katheter zu schaffen, der eine optimale Betriebssicherheit gewährleistet.

Die Aufgabe ist erfindungsgemäß dadurch gelöst, daß der Kunststoffschlauch mit einem weiteren Schlauch als Knickschutz umhüllt ist.

Vorzugsweise ist der innere Katheterschlauch aus Polyäthylen, Tetrafluorpolyäthylen oder Polyurethan und der äußere Knickschutzschlauch aus Silikon-Gummi oder einem anderen Elastomer gebildet. Diese Materialien weisen unterschiedliche mechanische Eigenschaften auf, wobei insbesondere Polyäthylen vergleichsweise hart und Silikon-Gummi vergleichsweise weich sind. Die Flexibilität wird also durch den äußeren umhüllenden Schlauch gewährleistet, wobei durch diesen Schlauch, der bei Abknickung des Katheters mögliche Biegeradius so vergrößert ist, daß ein Abbrechen bzw. Abknicken des inneren Schlauches verhindert wird.

Der äußere Schlauch hat im Normalzustand einen geringeren Innendurchmesser als der Außendurchmesser des inneren Katheterschlauches. Zum Aufbringen auf den Katheterschlauch wird der äußere Schlauch in einem organischen Lösungsmittel gequollen und im gequollenen Zustand auf den inneren Schlauch und das Anschlußstück aufgeschoben. Nach Verdunsten des Lösungsmittels schrumpft der äußere Schlauch auf Normalmaß und sitzt damit dicht und unter Spannung auf dem inneren Katheterschlauch sowie dem Anschlußstück.

Wird der Katheter insbesondere als Verbindung der extrakorporal tragbaren Dosiereinheit des Infusionsgerätes mit dem Patientenkörper verwendet, kann es vorteilhaft sein, daß allein der dem Anschluß zugewandte Bereich des Kunststoffschlauches mit dem weiteren Schlauch als Knickschutz umhüllt ist. Das heißt also, daß lediglich der außerhalb des Patientenkörper befindliche Teil des Katheters, der am meisten durch mechanische Beanspruchungen belastet ist, den Knickschutz aufweist, während subkutan nur der dünnere interne Schlauch verbleibt.

Ebensogut ist es aber auch möglich, daß der innere Kunststoffschlauch kürzer als der äußere Knickschutzschlauch ist. Ein solcher Katheter ist insbesondere für die Verwendung am oder im Bauchraum der Patienten geeignet, da der intreperitoneal verlegte Katheterabschnitt an seinem proximalen Ende möglichst weich sein soll.

Schließlich kann der innere Katheterschlauch auch über seine gesamte Länge von dem äußeren Knickschutzschlauch umgeben sein, wobei beide mit den Anschlüssen fest verbunden sind. Derartige Katheter können als sogenannte Zwischenstücke zur Verbindung einer Dosiereinheit mit einem Ausflußkatheter verwendet werden. Als Ausflußkatheter kann in einem solchen Fall beispielsweise wiederum ein erfindungsgemäßer Katheter verwendet werden; es sind aber auch andere übliche Katheter, beispielsweise sog. Butterfly-Katheter, anschließbar.

- 4 -    VPA 80 P 5116 E

Weitere Einzelheiten und Vorteile der Erfindung ergeben sich aus der nachfolgenden Figurenbeschreibung von Ausführungsbeispielen anhand der Zeichnung in Verbindung mit den Unteransprüchen. Es zeigen:

Fig. 1 einen ersten Katheter in Gesamtansicht,

Fig. 2 und 3 den zugehörigen weiblichen Luer-Anschluß in zwei verschiedenen Schnittdarstellungen,

Fig. 4 einen zweiten Katheter in Gesamtansicht, wobei die Anschlußbereiche jeweils geschnitten sind,

Fig. 5 und 6 den zugehörigen männlichen Luer-Anschluß in zwei verschiedenen Darstellungen sowie

Fig. 7 einen weiteren Katheter in Gesamtansicht, wobei der Katheterbereich teilweise geschnitten ist.

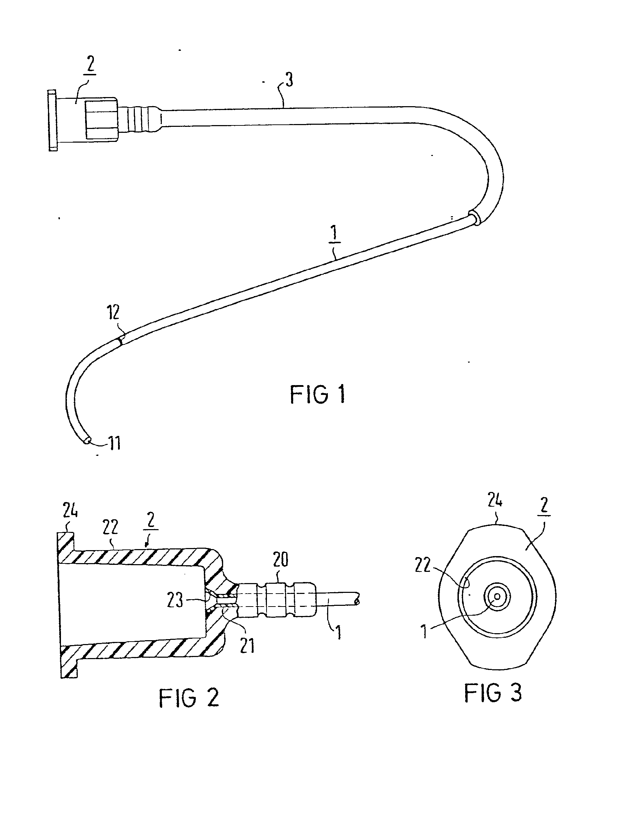

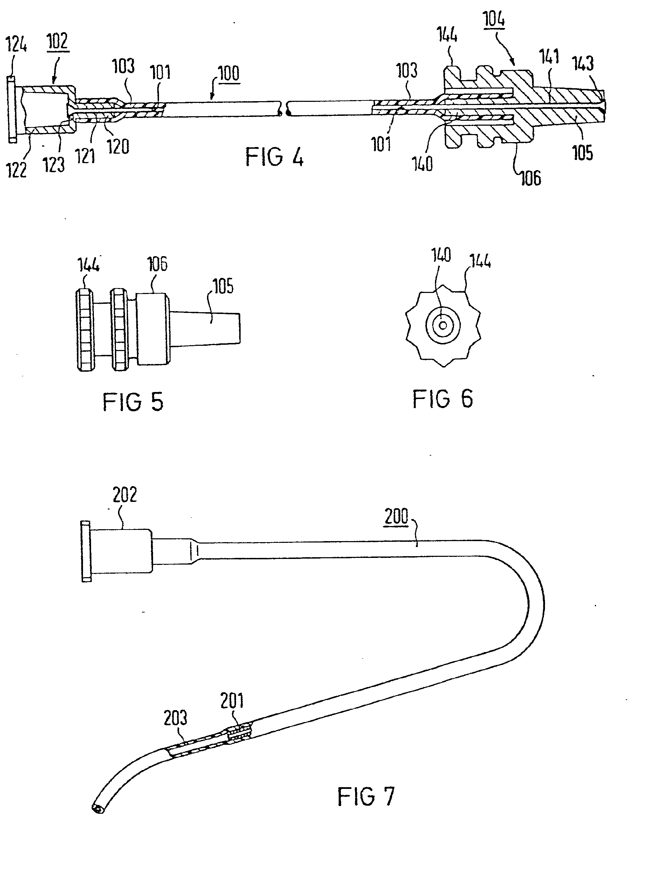

Die Katheter gemäß den Fig. 1 bis 3 sowie 7 werden als Ausflußkatheter verwendet, während der Katheter gemäß Fig. 4 bis 6 als Zwischenverbindung für einen Katheter entsprechend Fig. 1 bis 3 bzw. 7 benutzt werden kann.

In der Fig. 1 kennzeichnet 1 einen Katheterschlauch. Der Katheterschlauch 1 ist beispielsweise etwa 700 mm lang, hat einen Außendurchmesser von etwa 0,7 mm und ein inneres Lumen von ca. 0,3 mm. Das vordere, offene Ende 11 des Schlauches 1 ist mit seinen Kanten verrundet; ca. 50 mm vom proximalen Ende des Schlauches 1 entfernt ist eine röntgenkontrastgebende Markierung 12 als Applikationshilfe angebracht.

Der rückwärtige Teil des Schlauches 1 ist mit einem weiblichen Luer-Anschluß 2 verbunden. Solche Luer-Anschlüsse werden in der Medizintechnik üblicherweise für Verbindungen von Schlauch- und anderen Kathetern verwendet; sie lassen sich als Kunststoffspritzteile herstellen. Beim Herstellungsvorgang solcher Anschlüsse kann dabei der Kunststoffschlauch 1 unmittelbar dicht umspritzt werden.

Der rückwärtige Teil des Schlauches 1 ist von einem weiteren Schlauch 3 umgeben, der ein Innenlumen von ca. 0,5 mm und eine Wandstärke von ebenfalls 0,5 mm aufweist.

Aus den Figuren 2 und 3 ist der Aufbau eines üblicherweise verwendeten weiblichen Luer-Anschlußteils 2 im einzelnen ersichtlich. Ein zylindrisches Anschlußstück 20 weist auf der Oberfläche umlaufende Kerben auf. Ihnen befindet sich eine durchgängige Bohrung 21. Das Ansatzstück erweitert sich distal zu einem hohlzylindrischen Teil 22, das innen leicht konisch ausgestellt ist und einen männlichen Luer-Anschluß aufnehmen kann. Im Ansatzstück ist die Innenbohrung 21 mittels einer Kegelbohrung 23 an den größeren Umfang angepaßt. In die Bohrung 21 des Ansatzstückes 20 ist der Katheterschlauch eingepaßt. Zum Ende hin ist das Teil 22 mit einer Randausformung 24 versehen. In der frontständigen Sicht gemäß Fig. 3 ergeben sich Katheterschlauch 1 sowie die Teile 20 bis 24 im wesentlichen als konzentrische Ringe.

In der Fig. 4 ist der gesamte Verbindungskatheter mit 100 bezeichnet. Der Katheter 100 ist beispielsweise etwa 200 mm lang.

**0046485**

Aus den geschnittenen Teilen der Fig. 4 ist ersichtlich, daß der Verbindungskatheter 100 ebenfalls aus einem inneren Katheterschlauch 101 besteht, der über seine gesamte Länge von einem äußeren Knickschutzschlauch 103 umhüllt ist. An den Enden des Verbindungskatheters 100 befinden sich jeweils Luer-Anschlüsse, wobei 102 einen weiblichen Luer-Anschluß und 104 einen männlichen Luer-Anschluß bedeuten. Der weibliche Luer-Anschluß 102 entspricht im wesentlichen dem bereits oben beschriebenen.

Für den männlichen Luer-Anschluß 104 gilt aber weitgehend entsprechendes. Er besteht aus einem Konus 105 und einem daran distal angeordneten Griffteil 106. Das Griffteil 106 weist ein konzentrisches, freiliegendes Ansatzstück 140 auf, durch das ein durchgehender Kanal 141 in den Konus 105 führt. Der Kanal 141 erweitert sich wiederum am Ausgang des Konus 105 kegelförmig.

In der Fig. 7 ist der gesamte Katheter mit 200 bezeichnet. Dieser Katheter besteht entsprechend Fig. 1 und Fig. 4 aus einem inneren Schlauch 201 und einem äußeren Schlauch 203, die wie bereits oben beschrieben mit dem Ansatzstück eines weiblichen Luer-Anschlusses verbunden sind. Beim Katheter gemäß der Fig. 7 ist der eigentliche innere Katheterschlauch 101 kürzer als der äußere Schlauch 103. Die spezielle Verwendung eines solchen Katheters wird weiter unten noch beschrieben.

Bei den beschriebenen Ausführungsbeispielen besteht der innere Katheterschlauch 1, 101, 201 beispielsweise aus Polyäthylen-Material; solche Materialen können als Meterware bezogen und im jeweils geeigneten Maß abgeschnitten werden. Auch andere Kunststoffmaterialien, wie Tetrafluorpolyäthylen (bekannt unter dem Handelsnamen Teflon)

oder Polyurethan sind geeignet. Entscheidend ist, daß
das Kathetermaterial vergleichsweise hart ist und zum
Einführen in eine Körpervene oder -arterie geeignet ist.

Der Katheterschlauch ist jeweils dicht in die Durchbohrungen der zugehörigen Luer-Anschlüsse eingefügt und an
den kegelförmigen Ausstellungen fixiert. Dies kann - wie
erwähnt - durch Einspritzen, aber auch durch Einkleben
erfolgen. Durch die jeweils kegelförmige Ausstellung des
Kanals im Anschlußteil ist eine geeignete Fixierung möglich.

Der äußere Knickschutzschlauch besteht aus vergleichsweise weichem Material. Dabei ist es wesentlich, daß
innerer Schlauch und äußerer Schlauch insbesondere unterschiedliche Flexibilität aufweisen. Für den erwünschten
Zweck als Knickschutz ist daher Silikon-Gummi als Material für den äußeren Schlauch besonders geeignet. Der
Knickschutz wird im einzelnen dadurch bewirkt, daß der
äußere Schlauch stramm auf dem inneren Schlauch aufsitzt
und bei Abknickung des gesamten Katheters durch den
äußeren Schlauch der Biegeradius so vergrößert wird, daß
ein Abbrechen bzw. Abdichten des Innenschlauches verhindert wird.

Der Kunststoffschlauch als innerer Katheterschlauch aus
Polyäthylen sowie der äußere Knickschutzschlauch aus
Silikon-Gummi sind durch ihre mechanischen Eigenschaften,
insbesondere Flexibilität und Elastizität, gekennzeichnet. Dabei ist aber weiterhin wesentlich, daß der Innendurchmesser des äußeren Silikon-Schlauches im Normalzustand etwas kleiner ist als der Außendurchmesser des
inneren Polyäthylen-Katheters.

Zum Aufbringen des äußeren Schlauches auf den inneren
Katheterschlauch wird zunächst der Knickschutzschlauch
in einem organischen Lösungsmittel gequollen. Als geeignet hat sich hierfür beispielsweise Hexan erwiesen. Im gequollenen Zustand läßt sich dann der Silikon-Schlauch leicht auf den ersten Schlauch und die damit verbundenen Anschlußstücke des weiblichen und männlichen Luer-Anschlusses aufschieben. Nach Verdunsten des Hexans schrumpft der äußere Schlauch wieder auf Normalmaß; damit sitzt er dicht und unter Spannung auf dem inneren Katheterschlauch und den zugehörigen Anschlußstücken.

Die beschriebenen Katheter haben sich insbesondere zum Anschluß an die Dosiereinheit von extrakorporalen Infusionsgeräten als geeignet erwiesen. Dabei ist der Katheter gemäß Fig. 1 bis 3 insbesondere als Verbindung der Dosiereinheit direkt mit dem menschlichen Körper geeignet; das heißt im einzelnen, daß der von dem Knickschutzschlauch freie Teil des Katheters subkutan gelegt werden kann und beispielsweise in eine Vene eingeführt wird.

Der Katheter gemäß der Fig. 4 bis 6 ist dagegen als Zwischenverbindung von einer Dosiereinheit mit dem vorher beschriebenen Ausflußkatheter vorgesehen. Bei Infusionsgeräten werden solche Zwischenkatheter speziell dann verwendet, wenn die Anschlußstelle für einen Ausflußkatheter aus dem eigentlichen Infusionsgerät herausgelegt werden soll, um unterschiedlich ausgebildete Katheter anschließen zu können. Dies ist insbesondere dann von Bedeutung, wenn bei längerer Verwendung solcher Geräte wahlweise verschiedene Butterfly-Katheter anschließbar sein sollen.

Der Katheter nach Fig. 7 ist insbesondere für eine Verwendung im Bauchraum des Patienten geeignet. Es hat sich nämlich gezeigt, daß bei intraperitoneal gelegten Kathetern das vergleichsweise harte Polyäthylen-Material den Patienten unangenehm belästigen kann. Durch Umschließen des Katheterendes mit dem weichen Silikon-Gummi werden nun solche Belästigungen wirksam ausgeschlossen.

Durch die weiche Silikon-Ummantelung haben alle beschriebenen Katheter die günstigen Verwendungseigenschaften. In erster Linie werden solche Katheter für die betriebsmäßige Verwendung von extern am Körper tragbaren Infusionsgeräten verwendet. Daneben lassen sich solche Katheter natürlich auch bei implantierten Infusionsgeräten körperintern verwenden.

7 Figuren
14 Patentansprüche

Patentansprüche

1. Katheter zum Durchfluß von Flüssigkeiten, bestehend aus einem Kunststoffschlauch mit wenigstens einem distalen Anschluß, dadurch gekennzeichnet , daß der Kunststoffschlauch (1, 101, 201) mit einem weiteren Schlauch (3, 103, 203) als Knickschutz umhüllt ist.

2. Katheter nach Anspruch 1, wobei der Katheter insbesondere als Verbindung der Dosiereinheit eines Infusionsgerätes mit einem lebenden Körper dient, dadurch gekennzeichnet , daß lediglich der dem distalen Anschluß (2, 202) zugewandte Bereich des Kunststoffschlauches (1, 201) mit dem weiteren Schlauch (3, 203) als Knickschutz umhüllt ist.

3. Katheter nach den Ansprüchen 1 oder 2, dadurch gekennzeichnet , daß der innere Kunststoffschlauch (201) kürzer als der äußere Knickschutzschlauch (203) ist.

4. Katheter nach Anspruch 1, dadurch gekennzeichnet , daß der innere Kunststoffschlauch (101) über seine gesamte Länge vom äußeren Knickschutzschlauch (103) umgeben ist, welche beide mit den Anschlüssen (102, 104) fest verbunden sind.

5. Katheter nach Anspruch 1, dadurch gekennzeichnet , daß der Kunststoffschlauch (1, 101, 201) als innerer Katheterschlauch und der zusätzliche Schlauch (3, 103, 203) als äußerer Knickschutzschlauch unterschiedliche Flexibilität aufweisen.

0046485

6. Katheter nach Anspruch 5, d a d u r c h g e -
k e n n z e i c h n e t , daß der Kunststoffschlauch
(1, 101, 201) als innerer Katheterschlauch aus Polyäthylen, Tetrafluorpolyäthylen oder Polyurethan und der
Knickschutzschlauch (3, 103, 203) als umhüllender
Schlauch aus Silikon-Gummi oder anderen Elastomeren gebildet ist.

7. Katheter nach Anspruch 6, d a d u r c h g e -
k e n n z e i c h n e t , daß der äußere Knickschutzschlauch (3, 103, 203) einen geringeren Innendurchmesser
als der Außendurchmesser des inneren Katheterschlauches
(1, 101, 201) aufweist.

8. Katheter nach Anspruch 6 und 7, d a d u r c h
g e k e n n z e i c h n e t , daß der äußere Knickschutzschlauch (3, 103, 203) nach Quellung in einem
Lösungsmittel auf den Kunststoffschlauch (1, 101, 201)
größeren Durchmessers aufschiebbar ist.

9. Katheter nach Anspruch 1, d a d u r c h g e -
k e n n z e i c h n e t , daß der Kunststoffschlauch
(1, 101, 201) als innerer Katheterschlauch mit den Ansatzteilen (20, 120, 140) der Anschlüsse (2, 102, 104)
dicht verbunden, vorzugsweise in diese eingespritzt, ist.

10. Katheter nach Anspruch 1, d a d u r c h g e -
k e n n z e i c h n e t , daß die Anschlüsse (2, 102,
104, 202) Bestandteile von Luer- bzw. Luer-Lock-Verbindungen sind.

11. Katheter nach Anspruch 10, d a d u r c h g e -
k e n n z e i c h n e t , daß der äußere Knickschutzschlauch (103) auf das Ansatzteil (102) der weiblichen
Luer-Verbindung aufgezogen ist.

12. Katheter nach Anspruch 10, d a d u r c h   g e - k e n n z e i c h n e t ,   daß der männliche Luer-Anschluß (104) ein entsprechendes internes Ansatzteil (140) zum Aufziehen des äußeren Knickschutzschlauches (103) aufweist.

13. Katheter nach Anspruch 1, d a d u r c h   g e - k e n n z e i c h n e t ,   daß das proximale Ende (11) des Kunststoffschlauches (1) als innerer Katheterschlauch verrundet ist.

14. Katheter nach Anspruch 1, d a d u r c h   g e - k e n n z e i c h n e t ,   daß im Bereich des proximalen Teils des Kunststoffschlauches (1) als innerer Katheterschlauch eine röntgenkontrastgebende Markierung (12) angebracht ist.

FIG 1

FIG 2

FIG 3

0046485

FIG 4

FIG 5

FIG 6

FIG 7

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - C - 913 344 (W. RÜSCH) <br> * Ansprüche 1,2,3; Seite 2, Zeilen 28 bis 37, 46, 47, 58, 59, 63 bis 69; Fig. 2 * | 1,2, 9-11, 14 |
| | -- | |
| | DE - A1 - 2 708 213 (TSUTSUMI, MASATAKA) <br> * Seite 17, Zeilen 7 bis 18; Seite 18, Zeilen 17 bis 20; Fig. 2 * | 1,2 |
| | -- | |
| | DE - A1 - 2 909 430 (DOW CORNING K.K.) <br> * Ansprüche 1 bis 4, 6; Seite 10, Zeilen 4 bis 10, 21, 22; Seite 11, Zeilen 1 bis 12; Seite 12; Zeilen 9 bis 13; Fig. 3 * | 1,3,5, 6,9,10, 14 |
| | -- | |
| | FR - A - 1 445 705 (TECALEMIT) <br> * gesamtes Dokument * | 1,5-7 |
| | -- | |
| | DE - B - 1 264 182 (CONTINENTAL GUMMI-WERKE AG) <br> * gesamtes Dokument * | 1,5,6 |
| | -- | |
| | DE - A1 - 2 728 636 (OLYMPUS OPTICAL CO., LTD.) <br> * Ansprüche 1, 3; Seite 3, Zeilen 4 bis 10; Seite 4, Zeilen 29 bis 34 * | 1,6 |
| | -- | |
| | ./.. | |

**KLASSIFIKATION DER ANMELDUNG (Int. Cl.3)**

A 61 M  25/00

**RECHERCHIERTE SACHGEBIETE (Int. Cl.3)**

A 61 B   5/02
A 61 M   1/03
A 61 M  25/00
F 16 L  11/00
F 16 L  11/04

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X  Der vorliegende Recherchenbericht wurde für alle Patentansprüche erstellt.

| Recherchenort | Abschlußdatum der Recherche | Prüfer |
|---|---|---|
| Berlin | 16-11-1981 | CLOT |

## Europäisches Patentamt

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE – A1 – 2 553 100 (SHERWOOD MEDICAL INDUSTRIES) <br> * Ansprüche 1, 6; Seite 8, Zeilen 18 bis 22; Fig. 3 * | 6,14 |
| | DE – A1 – 2 543 486 (KENDALL CO.) <br> * Ansprüche 1, 5; Fig. 1 * | 9,10, 13 |
| | US – A – 4 052 990 (R.H. DODGSON) <br> * Anspruch 1; Fig. 4 * | 12 |
| A | FR – A – 2 041 652 (H. DURAND) <br> * Ansprüche 1, 4; Seite 1, Zeilen 24, 25, 35 bis 40; Seite 2, Zeilen 1 bis 12; Seite 4, Zeilen 4, 5; Fig. 4, 13 * | 1,2,4, 6,9, 10 |
| A | DE – A1 – 2 447 804 (SHERWOOD MEDICAL INDUSTRIES) <br> * Seite 2, Zeilen 12 bis 29; Seite 3, Zeilen 1, 2 * | 9,10 |

KLASSIFIKATION DER ANMELDUNG (Int. Cl.³)

RECHERCHIERTE SACHGEBIETE (Int. Cl.³)